(19)

Europäisches
Patentamt

European
Patent Office

Office européen
des brevets

(11)   EP 2 356 468 B1

(12)   **EUROPEAN PATENT SPECIFICATION**

(45) Date of publication and mention
of the grant of the patent:
**25.03.2015 Bulletin 2015/13**

(21) Application number: **08876030.1**

(22) Date of filing: **28.08.2008**

(51) Int Cl.:
***G01N 33/92*** *(2006.01)*

(86) International application number:
**PCT/IT2008/000560**

(87) International publication number:
**WO 2010/023698 (04.03.2010 Gazette 2010/09)**

(54) **METHOD FOR THE EVALUATION OF THE FUNCTIONAL STATUS OF CELL MEMBRANES REFERRED TO THE FATTY ACID COMPONENTS**

VERFAHREN ZUR BEWERTUNG DES FUNKTIONELLEN STATUS VON ZELLMEMBRANEN MIT BEZUG AUF DIE FETTSÄUREKOMPONENTEN

PROCÉDÉ POUR L'ÉVALUATION DE L'ÉTAT FONCTIONNEL DE MEMBRANES CELLULAIRES ATTRIBUÉ AUX COMPOSANTS D'ACIDES GRAS

(84) Designated Contracting States:
**AT BE BG CH CY CZ DE DK EE ES FI FR GB GR HR HU IE IS IT LI LT LU LV MC MT NL NO PL PT RO SE SI SK TR**

(43) Date of publication of application:
**17.08.2011 Bulletin 2011/33**

(73) Proprietor: **Lipinutragen S.R.L.**
**40129 Bologna (IT)**

(72) Inventor: **FERRERI Carla**
**40125 Bologna (IT)**

(74) Representative: **Brighenti, Livio et al**
**NOTARBARTOLO & GERVASI S.p.A.**
**Corso di Porta Vittoria, 9**
**20122 Milano (IT)**

(56) References cited:
• **FERRERI CARLA ET AL: "Trans fatty acids and atopic eczema/dermatitis syndrome: The relationship with a free radical cis-trans isomerization of membrane lipids" LIPIDS, vol. 40, no. 7, July 2005 (2005-07), pages 661-667, XP002514982 ISSN: 0024-4201**
• **LEIDL KATHARINA ET AL: "Mass spectrometric analysis of lipid species of human circulating blood cells" BIOCHIMICA ET BIOPHYSICA ACTA, vol. 1781, no. 10, 6 August 2008 (2008-08-06), pages 655-664, XP002514983 ISSN: 1388-1981**
• **DING J ET AL: "Application of the accurate mass and time tag approach in studies of the human blood lipidome" JOURNAL OF CHROMATOGRAPHY B: BIOMEDICAL SCIENCES & APPLICATIONS, ELSEVIER, AMSTERDAM, NL, vol. 871, no. 2, 15 August 2008 (2008-08-15), pages 243-252, XP023782700 ISSN: 1570-0232 [retrieved on 2008-05-07]**

Printed by Jouve, 75001 PARIS (FR)

**Description**

**Field of the invention**

The application refers to the field of diagnostic methods, in particular methods for the evaluation of the cell membranes status.

**State of the art**

The use of lipid analysis in health is well documented. Literature as well as websites (see www.lipidlibrary.co.uk/lit surv/general/reviews2.htm) are full of records on this interdisciplinary subject that links chemistry to biology and medicine. The usefulness of lipid analysis in health has become more and more evident and the discipline of lipidomics has recently gathered all investigations on lipids and their transformations, moreover it is also acknowledged that the lipid organisation can be influenced by the diet as well as by physiological and pathological conditions (see Maxfield F.R. et al. - Role of cholesterol and lipid organisation in disease - Nature 2005, 438, 612).
Lipid composition and lipid markers in health and diseases are studied by functional lipidomics with applicability to medicine as diagnostic tools.
Fatty acids can be followed up in various biological compartments, for example in blood plasma as free fatty acids (FFA) and triglycerides (TG), as well as in cell membranes, where they form the hydrophobic part of the phospholipids structures (PL).
Focusing the interest on cell membrane lipids, the protocols to isolate, extract and evaluate quantitatively and qualitatively the fatty acid residues of phospholipids are very well known (see for example: Bligh E.G. et al. - A rapid method of total lipid extraction and purification - Can. J. Biochem. Physiol. 1959, 37, 911 and Kramer J. K. G. et al. Evaluating acid and base catalysts in the methylation of milk and rumen fatty acids with special emphasis on conjugated dienes and total trans fatty acids - Lipids 1997, 32, 1219). The most widely used analytical method for fatty acid analysis is gas chromatography (GC). The GC analysis is performed on fatty acid methyl esters under known conditions and is the most efficient method allowing for the separation of all classes of fatty acids, namely saturated, unsaturated, cis and trans isomers. The molecular library of geometrical trans isomers to be used as reference in the GC analysis has been obtained by isomerization of natural oils, and their application to the identification of these isomers in biological samples has been described (Abdelouahid Samadi et al. - JAOCS 2004, Vol. 81, N° 8, P. 753-758; see also IT 1.327.120). It is worth underlining that the naturally occurring cis configuration of fatty acids is provided enzymatically, whereas the trans isomers can be considered unnatural for humans; the formation of trans geometrical isomers has been correlated to radical stress and several diseases that include the production of free radicals have been individuated.
In functional lipidomics, the fatty acid analysis is used for medical applications, because it is possible to make a comparison between healthy status and pathological conditions, thus individuating some abnormalities of the fatty acid levels, either expressed as relative percentages or as absolute concentration as derived from the GC analysis.
The fatty acid composition of various tissues is well known, and the healthy controls furnish a reference group of normal ranges in respect of which the abnormal values can be identified.
Moreover, in lipidomics it has been established that fatty acids are not only important as structural units of cell membranes, but also as signaling molecules, that govern several biochemical pathways, including inflammation and apoptosis. The methods for lipid analysis currently in use consist of a series of steps, as follows: (a) measuring a quantitative feature of a fatty acid (or a different type of lipids) within a sample (erythrocyte, plasma, leukocyte, etc) derived from a subject, either as absolute value (against an internal references) or as relative percentage of the whole chromatographed peaks; (b) comparing the measurement obtained in step (a) with the normal values taken either from the literature and/or from the group of healthy controls, (c) if the measurement is significantly different from the threshold value, the measurement is indicative that the subject has or is at risk of developing the disease.
This approach is also described, for example in the following patens US - 2005/009005 and US - 5,075, 101.
According to another method the lipid metabolite data are quantitatively and/or comparatively evaluated, and their values constitute the so-called metabolomic profile, that is indicative of metabolic status.
As previously described, the method consists in the measurements of the quantity of one or more lipid biomarkers; profiles or ratios of such markers can provide the likelihood or progression of diseases or health conditions, such as weight gain or weight loss, growth or wasting, obesity, diabetes, and aging.
One of these methods (see US - 5,075, 101) allows the measure of various indexes like Fatty Acids and Essential Fatty Acid (EFA) Deficiencies (EFAD), which evaluate lipid and fatty acid biochemical status, by the analysis of tissues of a test subject, comparing patterns or domains between normal and abnormal biochemistry, for the purposes of diagnosing abnormalities.
Ferreri et al. (Lipids 40(7), 661-667, 2005) discloses the comparison of phospholipids in isolated erythrocyte membranes of children affected by atopic eczema/dermatitis syndrome and healthy children. A panel of major FA and trans FA were compared.

The effect of fatty acids can be considered also under other aspects (see Gunstone F.D. - Fatty acids and Lipid Chemistry, Aspen Publishers; Gaithersburg, 1999) such as their chemical and biochemical reactivity. In particular three features are important:

1) the sensitivity of unsaturated fatty acids to oxidative and free radical processes;
2) the contribution of unsaturated fatty acids as elements for membranes fluidity and permeability;
3) the involvement in biochemical pathways such as inflammation (in particular for the omega-6 fatty acid, namely arachidonic acid).

These aspects are taken into account using the well known indexes that come from the values of the fatty acid analysis expressed as relative percentages in different samples. They can be calculated not only in membranes but also in plasma and other tissues:

- unsaturation index UI, obtained as follows: 1 x (%MUFA) + 2 x (% dienoics) + 3 x (% trienoics) + 4 x (% tetraenoics) + 5 x (% pentaenoics) + 6 x (% hexaenoics).
- Peroxidation index PI, obtained as follows: PI = [(% MUFA x 0.025) + (% dienoic x 1) + (% trienoic x 2) + (% tetraenoic x 4) + (% pentaenoic x 6) + (% hexaenoic x 8)]
- Omega-6/omega-3 ratio, calculated either from the ratio arachidonic acid/EPA or by considering all the omega-6 and omega-3 fatty acids present in the analysis.

As it can be seen in the light of the above reported state of the art there is no record of only one index capable of giving an indication of the whole membrane status, summarising the contribution to the membrane - from the biochemical and biophysical points of view - of all fatty acid categories and indexes, while, on the other hand it is evident how such an index would be useful for establishing the unbalance among the different types, families and ratios of fatty acids (including the effect of radical stress).

**Brief description of the Figure**

The enclosed figure shows schematically the various step of the process according to the invention.

**Summary of the invention**

The invention refers to a method for the global evaluation of the status of cell membranes as defined in the claims.

**Detailed description of the invention**

The present invention allows to make a step forward in the lipid analysis of cell membranes and makes available a method for a global evaluation of the status of cell membranes related to the fatty acid components in order to have a comprehensive information of the health and quality of life of the examined patient.

The method offers an innovative procedure for the conversion of the data coming from the fatty acid analysis of membrane phospholipids, the latter being done by known procedures. It is specifically applied to cell membranes, because the interval values that are considered are referred to the various fatty acids at the level of membrane phospholipids, including saturated and cis unsaturated fatty acids, as well as trans fatty acid isomers obtained from a molecular library related to free radical stress.

The method can be applied to blood cell membranes (erythrocyte, lymphocyte, platelets) and can be valid also for other tissues or cells.

For practical reasons the description and examples reported hereinafter will be referred to erythrocyte membranes.

According to the invention method the erythrocyte membrane fatty acid composition described as relative percentages of the whole chromatographed peaks is initially determined on a sample of patient blood.

Thereafter the obtained values are compared with the standard erythrocyte membrane fatty acid composition described as intervals of values (minimum-maximum) that are well known from the literature (see Lauritzen L. et al. - The essentiality of long chain n-3 fatty acids in relation to development and function of the brain and retina - Progr. Lipid Res. 2001, 40,1).

To proceed with the evaluation of the global membrane status, the method presents Table of Conversion and Combination factors that are used in subsequent steps to transform only the abnormal values (i.e. those falling out of the above said standard intervals) found in the sample into percentages, expressing the perturbation of the fatty acid balance in the membrane sample, this will lead to generate the final index MUI (Membrane Unbalance Index).

The method allows personalized health care, connecting the MUI to personal information of the subject on familial, nutritional and metabolic conditions. This allows to evaluate the value of MUI in humans under different physiological and pathological conditions, that includes the evaluation of free radical stress involving the geometry of fatty acids. MUI gives a new meaning to the effects of fatty acid decrease or increase because it focuses on the global conditions of the membranes and the balance of their structural and functional roles. Several other cells and tissues, whose standard composition is known, can be examined by this method.

The index MUI obtained by the invention's method expresses the distress of a membrane due to the impairment of the natural and optimal lipid balance, that can not be connected immediately to a disease. In this case the MUI represents also a tool for preventive medicine and individuates a targeted prevention therapy based on the membrane balance restoring.

Moreover the method according to the invention allows the evaluation of the effects of nutraceuticals or natural therapies on the membrane unbalance.

In fact, it is known that several therapeutic and natural medical treatments can have influence on the membrane fatty acid composition and change the different components of fatty acid families. With MUI, it will be possible to evaluate whether the changes of fatty acids affected the membrane balance in a positive manner, focusing on global effects and indicating synergic strategies among the different classes of lipids.

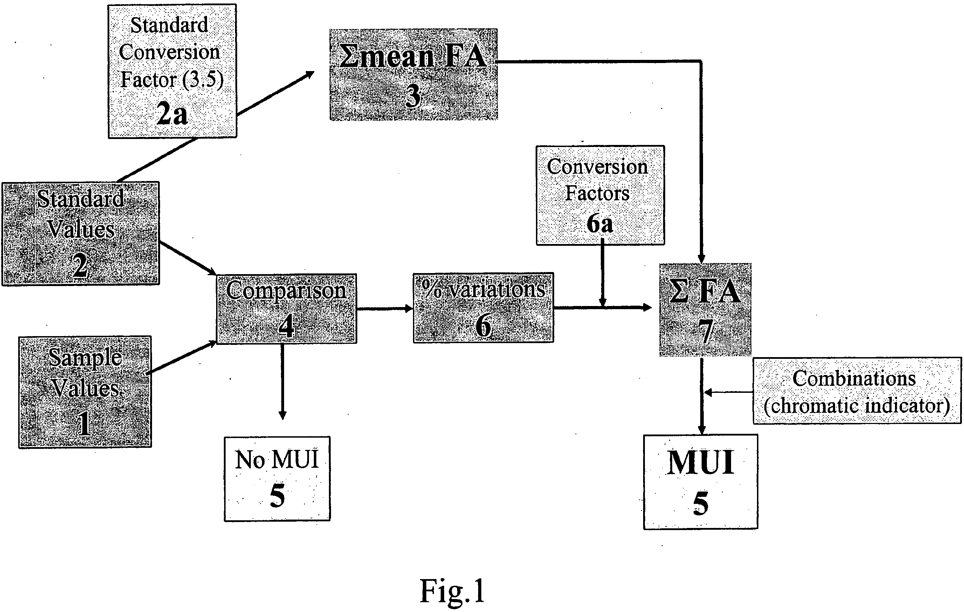

The method according to the invention comprises various steps which are schematically represented in Fig. 1.

As shown in Figure 1 in the first step (Samples Analysis) 1 samples of patient's blood are collected and analyzed according to known techniques in order to establish the quantities of the following 19 items:

a) Palmitic acid (16:0)
b) Stearic acid (18:0)
c) Palmitoleic acid (9cis-16:1)
d) Oleic acid (9cis-18:1)
e) Elaidic acid (9trans-18:1) + Trans-vaccenic acid (11trans-18:1) expressed together as Trans-18:1
f) Vaccenic acid (11cis-18:1)
g) Linoleic acid (9cis,12cis-18:2)
h) Eicosatrienoic acid (8cis,11cis,14cis-20 :3)
i) Arachidonic acid (5cis,8cis, 11cis,14cis-20 :4)
l) Trans isomers of arachidonic acid (mixture of mono-trans isomers of arachidonic acid: 5cis,8cis,11cis,14trans-20:4 + 5cis,8cis,11trans,14cis-20:4 + 5cis,8trans,11cis,14cis-20:4 + 5trans,8cis,11cis,14cis-20:4)
m) Eicosapentaenoic acid (5cis,8cis,11cis,14cis,17cis-20:5)
n) Docosahexaenoic acid (4cis,7cis,10cis,13cis,16cis,19cis-22:6)
o) Total trans fatty acids (TFA) (the sum of the trans geometrical isomers 18:1 and 20:4)
p) Total SFA (the sum of saturated fatty acids 16:0 + 18:0)
q) Total MUFA (the sum of monounsaturated fatty acids 16:1 + cis 18:1)
r) Total PUFA (the sum of polyunsaturated fatty acids 18:2, 20:3, 20:4, 20:5, 22:6) -
s) Ratio between Saturated Fatty Acids and Monounsaturated Fatty Acids (SFA/MUFA)
t) Ratio between the sum of omega-6 (linoleic acid + eicosatrienoic acid + arachidonic acid) and the sum of omega-3 (eicosapentaenoic acid + docosahexaenoic acid) (OMEGA-6/OMEGA-3)
u) Ratio between linoleic acid and stearic acid (EFA deficiency)

The obtained values for each of the above said items are compared 4 with the Standard Fatty acid contents (Standard Values) 2 for the same kind of cell and the percentage variation of the measured values, in respect of the corresponding inferior or superior limit of the Standard range is calculated 6.

The Standard Fatty acid contents is known as the values referred to the normality; in particular for erythrocytes membranes the average interval values derived from healthy control groups are very well represented in literature and are reported in Table 1.

**TABLE 1**

| FATTY ACIDS | Normal intervals (%rel.) | Conversion Factors BASIC | |
|---|---|---|---|
| | | -%var | +%var |
| Palmitic (16:0) | 17-27 | 1 | 23 |
| Palmitoleic (16:1) | 0.2 - 0.5 | 1 | 13 |

(continued)

| FATTY ACIDS | Normal intervals (%rel.) | Conversion Factors BASIC | |
|---|---|---|---|
| | | -%var | +%var |
| Stearic (18:0) | 13-20 | 23 | 2 |
| Oleic (18:1) | 9-18 | 1.7 | 1 |
| Trans 18:1 (9t+11t-18:1) | <0.3 | 0 | 0 |
| Vaccenic (18:1) | 0.7-13 | 0 | 0 |
| Linoleic (18:2) (omega-6) | 9-16 | 23 | 23 |
| Eicosatrienoic (20:3) (omega-6) | 1.9-2.4 | 2 | 2 |
| Arachidonic (20:4)(omega-6) | 13-17 | 2 | 23 |
| Mono trans ARA | <0.3 | 0 | |
| EPA (20:5) (omega-3) | 0.5-0.9 | 2 | 13 |
| DHA (22:6) (omega-3) | 5-7 | 2 | 13 |
| Tot. saturated (SFA) | 30-45 | 13 | 23 |
| Tot. monounsaturated (MUFA) | 13-23 | | 13 |
| Tot. polyunsatured (PUFA) | 28-39 | 23 | 2.3 |
| Tot. trans fatty acids (TFA) | <0.3 | 0 | 23 |
| SFA/MUFA ratio | 1.7-2 | 0 | 23 |
| Omega-6/ omega-3 ratio | 3.5-5.5 | 0 | 13 |
| EFA deficiency | >0.4 | 0 | 1 |

If the values measured of each item as above defined are included in the corresponding ranges as reported in the Standard Table there is no necessity of calculating the MUI **5** since the status of the cell membrane as a whole can be considered satisfactory.

Thereafter the value obtained as described above is multiplied by an arbitrary number representing the Basic Conversion Factor, which is represented by the conversion factors reported in Tables 1 - 4, and the so obtained absolute values are summed to represent the total percentage of unbalance (E FA).

The minimum and maximum Standard values of each item is multiplied by an arbitrary number, which is a Standard Conversion Factor and the two limit values are mediated giving the $\Sigma$ mean FA value representing the contributions of the elements of the standard membrane fatty acid analysis.

Finally the all comprehensive index expressing the total membrane cell unbalance MUI is calculated according to the following formula

$$MUI = (\Sigma \ FA) \ x \ 100 / \ \Sigma \ mean \ FA$$

The above said arbitrary number representing the Basic Conversion Factor is represented by the conversions factors reported in the following

For example, as it can be seen in Table 1 the range of oleic acid is 9 -18; if the value of oleic acid found in the patient's blood is 19.9%, it will be 1.9 more than the maximum value of the range. This corresponds to a difference of 10.56% from the normal values (1.9 : x = 18 : 100 ; x = 10.56%) with the positive sign + because is an increment. If the value of oleic acid is 8.1%, it is 0.9 less than the minimum value of the normal range. This corresponds to a 10% decrement, therefore with negative sign.

Such increment or decrement for each one of the 19 items specified above is thereafter multiplied by an arbitrary number (Basic Conversion Factor) **6a**; in Table 1 are reported examples of possible Basic Conversion Factors for each item.

To go back to the reported example, if the excess for oleic acid is +10.56% its contribution to the unbalance of the membrane under examination is 10.56 (absolute value, i.e. without plus or minus sign) since the corresponding (+%var.) Basic Conversion Factor is 1.

The sum of the absolute values calculated as above described represents the total percentage of unbalance (E FA) 7 of the membrane in the sample, this is also referred as Basic FA Contribution in contrast with other FA contributions that will be explained hereinafter.

This number will be different depending on the values reported in each analysis; only samples having the same values of the 17 items, will have the same numbers therefore this number can be considered a sort of finger print of the membrane status

At the same time, the E mean FA **3** is calculated as follows.

The minimum and maximum Standard values of each item is multiplied by an arbitrary number (Standard Conversion Factor) **2a**, according to the example the chosen value is 3.5, and two limit values are obtained (one for the minimum and one for the maximum):

$$\Sigma(\text{mean FA max}) = 225 \times 3.5 = 790.65$$

$$\Sigma(\text{mean FA min}) = 168.9 \times 3.5 = 591.15$$

from which the wanted $\Sigma$ mean FA results as: 790.65 + 591.15/2 = 690.9

This value represents the value summarising the contributions of the elements of the standard membrane fatty acid analysis, and it will used by the method in the subsequent steps.

Starting from a different table of analytical data, for example in case of other types of tissues or cells, it is obvious that the $\Sigma$ mean FA will change accordingly..

Now, going back to the above calculated $\Sigma$ FA the wanted all comprehensive index expressing the total membrane cell unbalance (MUI) is given by the following formula:

$$\text{MUI} = (\Sigma \text{ FA}) \times 100 / \Sigma \text{ mean FA}$$

Considering the above reported example, referring to the unique variation regarding the oleic acid, it will be:

$$\text{MUI} = 10.56 \times 100/690.9 = 1{,}53\%$$

Moreover, as said above, the method allows a more precise, practically personalized, diagnosis when more information about the patients or the familial history is available.

In fact, as shown above, to calculate the membrane index, the percentages in excess or defect of the sample values are multiplied for the corresponding Basic Conversion Factors (for example those reported in Table 1) in order to evaluate the contribution to the unbalance as the sum of said products.

However instead of the Basic Conversion Factors considered above (for example those reported in Table 1) in view of the above said information on the health status of the patient or his relatives, other Conversion Factors can be listed, the value of which is in any case chosen in the interval between 0 and the Standard Conversion Factor used for the calculation of the $\Sigma$ mean FA (in the case reported as example these Conversion Factors are comprised between 0 and 3.5).

The values attributed to the conversion factors in this case specifically take into consideration the importance of each item for the variation of membrane properties and its capacity of acting in the metabolism at level of cell signaling, and consequently their importance for the predictability of the possible onset of diseases, aging and stress. The knowledge of the role played by fatty acids in membranes in respect of various diseases can be found in any basic publications and books on biochemistry and lipidomics helping in building up the desired Tables of Conversion Factors.

Tables 2, 3 and 4 are examples which show other conversion factors identified by the corresponding names that suggest their field of application in diagnosis (respectively cardio, stress and UFA deficit).

**TABLE 2**

| FATTY ACIDS | Conversion Factors CARDIO | |
|---|---|---|
| | -%var | +%var |
| Palmitic (16:0) | 1 | 3.5 |

(continued)

| FATTY ACIDS | Conversion Factors CARDIO | |
| --- | --- | --- |
| | -%var | +%var |
| Palmitoleic (16:1) | 2 | 1 |
| Stearic (18:0) | 2 | 4 |
| Oleic (18:1) | 2 | 15 |
| Trans 18:1 (9t+11t-18:1) | 0 | 3 |
| Vaccenic (18:1) | 2 | 1 |
| Linoleic (18:2) (omega-6) | 2.5 | 3 |
| Eicosatrienoic (20:3)(omega-6) | 3 | 3 |
| Arachidonic (20:4)(omega-6) | 3 | 3.5 |
| Mono trans ARA | 1 | 3 |
| EPA (20:5) (omega-3) | 3 | 1.5 |
| DHA (22:6)(omega-3) | 3 | 1 |
| Tot. satured (SFA) | 1.5 | 3.5 |
| Tot. monounsatured (MUFA) | 3.5 | 1.5 |
| Tot. polyunsatured (PUFA) | 3.5 | 1 |
| Tot. trans fatty acids (TFA) | 0 | 3 |
| SFA/MUFA ratio | 2 | 35 |
| Omega-6/ omega-3 ratio | 25 | 35 |
| EFA deficiency | 2 | 3 |

**TABLE 3**

| FATTY ACIDS | Conversion Factors UFA deficit | |
| --- | --- | --- |
| | -%var | +%var |
| Palmitic (16:0) | 15 | 3 |
| Palmitoleic(16:1) | 2.5 | 1 |
| Stearic (18:0) | 2.5 | 3 |
| Oleic (18:1) | 2 | 1 |
| Trans 18:1 (9t+11t-18:1) | 1 | 3 |
| Vaccenic (18:1) | 1 | 1 |
| Linoleic (18:2)(omega-6) | 3 | 1.5 |
| Eicosatrienoic (20:3)(omega-6) | 3 | 3 |
| Arachidonic (20:4)(omega-6) | 2.5 | 3.5 |
| Mono trans ARA | 1 | 3 |
| EPA (20:5) (omega-3) | 3.5 | 2 |
| DHA (22:6)(omega-3) | 35 | 2 |
| Tot. saturated (SFA) | 1 | 3 |
| Tot. monounsaturated (MUFA) | 2 | 1 |

(continued)

| FATTY ACIDS | Conversion Factors UFA deficit | |
| --- | --- | --- |
| | -%var | +%var |
| Tot. polyunsatured (PUFA) | 3 | 1 |
| Tot. trans fatty acids (TFA) | 1 | 3 |
| SFAIMUFA ratio | 2 | 3.5 |
| Omega-6/ omega-3 ratio | 1.5 | 3 |
| EFA deficiency | 3 | 1 |

TABLE 4

| FATTY ACIDS | Conversion Factors STRESS | |
| --- | --- | --- |
| | -%var | +%var |
| Palmitic (16:0) | 1 | 3 |
| Palmitoleic (16:1) | 2.5 | 1 |
| Stearic (18:0) | 2 | 3 |
| Oleic (18:1) | 2 | 2 |
| Trans 18:1 (9t+11t-18:1) | 0 | 3.5 |
| Vaccenic (18:1) | 1 | 2 |
| Linoleic (18:2) (omega-6) | 3 | 2 |
| Eicosatrienoic (20:3)(omega-6) | 3 | 2 |
| Arachidonic (20:4)(omega-6) | 2 | 3.5 |
| Mono trans ARA | 0 | 35 |
| EPA (20:5) (omega-3) | 3.5 | 1 |
| DHA (22:6)(omega-3) | 3.5 | 1 |
| Tot. saturated (SFA) | 1 | 3 |
| Tot. monounsatured (MUFA) | 1 | 2 |
| Tot. polyunsaturated (PUFA) | 3 | 1 |
| Tot. trans fatty acids (TFA) | 1 | 3.5 |
| SFA/MUFA ratio | 1 | 35 |
| Omega-6/ omega-3 ratio | 15 | 3.5 |
| EFA deficiency | 3 | 1 |

According to a particular embodiment of the invention the indication of the MUI can be coupled with a chromatic and/or symbolic indicator that reports in a graphic and immediate way the sample membrane status of unbalance.

In fact, the color can for example be in relation with the quantity and type of the items that are out of ranges and the corresponding degree of unbalance that they cause to membranes.

For example, choosing green, yellow and red as a three colors scale for indicating increasingly gravity of the membrane unbalance, in the case of only one item unbalanced (oleic acid + 10.56%) (see the example) the MUI of 1.53% will have a chromatic indicator that is green, because oleic acid excess can clearly derive from the dietary contribution of olive oil, and is giving an optimal contribution to the membrane status.

There can be several Combinations sets referred to different groups of fatty acid values and other items of the analysis, that can be out of ranges (positively or negatively).

Table 5 represents the main combinations and the corresponding chromatic indicator. The invention obviously comprises all the combinations that can be added on the basis of the increasing knowledge on the membrane balance.

In order to simplify the operations a computer-assisted procedure can be figured out, wherein the tabulated data and the conversion factors are inserted in a program that calculates the index, and then combines the index with the chromatic color. The computer-assisted tabulation can help to create a data base for lipid analysis based on the membrane status index for further use in medicine and epidemiology.

TABLE 5 - Possible Combinations and corresponding chromatic indicators

| FATTY ACIDS | COMBINATIONS | | | |
|---|---|---|---|---|
| | green | yellow | red | red |
| Palmitic (16:0) | | | +%var | |
| Palminoleic (16:1) | | +%var | | |
| Stearic (18:0) | | | -%var | |
| Oleic (18:1) | +%var | +%var | | |
| Trans 18:1 | | | | |
| Vaccenic (18:1) | | +%var | | |
| Linoleic (18:2) (omega-6) | | | | |
| Eicosatrienoic (20:3) (omega-6) | | -%var | | -%var |
| Arachidonic (20:4)(omega-6) | | | | +%var |
| Mono trans ARA | | | | |
| EPA (20:5) (omega-3) | | | | |
| DHA (22:6) (omega-3) | | | -%var | |
| Tot. saturated (SFA) | | | +%var | |
| Tot. monounsaturated (MUFA) | +%var | | | |
| Tot. polyunsaturated (PUFA) | | -%var | | +%var |
| Tot. trans fatty acids (TFA) | | | | +%var |
| SFA/MUFA ratio | | -%var | +%var | |
| Omega-6/ omega-3 ratio | | | | +%var |
| EFA deficiency | | | | |

The following examples illustrate the present invention more clearly:

EXAMPLE 1

Table 6 shows the values of a fatty acid analysis from erythrocyte membranes of a human subject with the % variations in positive and negative; by executing the steps indicated in the method, the $\Sigma$ FA value increments by 104.06 compared to $\Sigma$ mean FA = 690.9. The index is 15.1%.

In case that the patient from which the sample is collected (or members of his family) suffers of cardiovascular diseases the CARDIO configuration of Table 2 can be applied, that gives the $\Sigma$ FA value increments of 167.80 and the index of 24.3%. The increase of the index underlines that the unbalance for this subject is an indicator of the cardiovascular district.

According to Table 5 the presence of altered levels of arachidonic acid (positive), omega-6/omega-3 ratio (positive)and eicosatrienoic acid (negative), will give the chromatic indicator as RED.

Table 6 - Example n. 1

| FATTY ACIDS | Normal intervals (%rel.) | Found values (%rel.) | %var |
|---|---|---|---|
| Palmitic (16:0) | 17-27 | 26 | |

(continued)

| FATTY ACIDS | Normal intervals (%rel.) | Found values (%rel.) | %var |
|---|---|---|---|
| Palmitoleic (16:1) | 0.2-0.5 | 0.3 | |
| Stearic (18:0) | 13-20 | 17.6 | |
| Oleic (18:1) | 9-18 | 18 | |
| Trans 18:1 | <0.3 | 0.1 | |
| Vaccenic (18:1) | 0.7-1.3 | 1.4 | + 7.69% |
| Linoleic (18:3)(omega-6) | 9-16 | 11.4 | |
| Eicosatrenoic (20:3)(omega-6) | 1.9-2.4 | 1.6 | -15.79% |
| Arachidonic (20:4)(omega-6) | 13-17 | 18.4 | +8.24% |
| Mono trans ARA | <0.3 | 0.1 | |
| EPA (20:5) (omega-3) | 0.5-0.9 | 0.4 | -20.00% |
| DHA (22:3)(omega-3) | 5-7 | 4.6 | -8% |
| Tot. saturated (SFA) | 30-45 | 43.6 | |
| Tot. monounsaturated (MUFA) | 13-23 | 19.7 | |
| Tot. polyunsaturated (PUFA) | 28-39 | 2.3 | |
| Tot. trans fatty acids (TFA) | <0.3 | 0.2 | |
| SFA/MUFA ratio | 1.7-2 | 2.2 | +10.00% |
| Omega-6/ omega-3 ratio | 3.5-5.5 | 6.2 | +12.73% |
| EFA deficiency | >0.4 | 0.6 | |

EXAMPLE 2

Table 7 shows the values of a fatty acid analysis from erythrocyte membranes of a human subject with the % variations in positive and negative. According to the BASIC configuration of Table 1, and executing the steps indicated in the method, the S (sample FA) value increments by 344.50 compared to S (mean FA) = 690.9. The index is 49.9%. In case that the sample is connected to a human subject where the stress is denoted, the STRESS configuration of can be applied, that gives the S (sample FA) value increments of 552.39 that gives an MUI of 79.9%. The increase of the index underlines the stress component in the membrane unbalance. According to Table 5 the presence of altered levels of arachidonic acid (positive), SFA/MUFA ratio (positive) and trans isomers (positive), will give the chromatic indicator as RED

**Table 7 - Example n. 2**

| Fatty ACIDS | Normal intervals (%rel.) | Found values (%rel.) | %var |
|---|---|---|---|
| Palmitic (16:0) | 17-27 | 26 | |
| Palmitoleic (16:1) | 0.2 - 0.5 | 0.6 | + 20.00% |
| Stearic (18:0) | 13-20 | 14.4 | |
| Oleic (18:1) | 9-18 | 17 | |
| Trans 18:1 | <0.3 | 0.4 | +33.33% |
| Vaccenic (18:1) | 0.7-1.3 | 1.2 | |
| Linoleic (18:2)(omega-6) | 9-16 | 11.7 | |
| Eicosatrienoic (20:3)(omega-6) | 1.9-2.4 | 2.1 | |
| Arachidonic (20:4)(omega-6) | 13-17 | 17.8 | +4.71% |

(continued)

| Fatty ACIDS | Normal intervals (%rel.) | Found values (%rel.) | %var |
|---|---|---|---|
| Mono trans ARA | <0.3 | 0.2 | |
| EPA (20:5) (omega-3) | 0.5-0.9 | 0.8 | |
| DHA (22:6)(omega-3) | 5-7 | 7.8 | +11.43% |
| Tot. saturated (SFA) | 30-45 | 40.4 | |
| Tot. monounsaturated (MUFA) | 13-23 | 18.8 | |
| Tot. polyunsaturated (PUPA) Tot. polyunsaturated (PUFA) | 28-39 | 40.1 | + 2.82 % |
| Tot. trans fatty acids (TFA) | <0.3 | 0.6 | +100.00% |
| SFA/MUFA ratio | 1.7-2 | 2.2 | + 10.00% |
| Omega-6/omega-3 ratio | 3.5 - 5.5 | 3.7 | |
| EFA deficiency | >0.4 | 0.8 | |

**Claims**

1. Method for the global evaluation of the status of cell membranes related to its fatty acid components, wherein said fatty acid are membrane phospholipids and said cells are blood cells, comprising the following step:

   - samples of patient's blood are analyzed according to known techniques in order to establish the quantities of the following 19 items:

      a) Palmitic acid (16:0)
      b) Stearic acid (18:0)
      c) Palmitoleic acid (9cis-16:1)
      d) Oleic acid (9cis-18:1)
      e) Elaidic acid (9trans-18:1) + Trans-vaccenic acid, 11trans-18:1, expressed together as Trans-18:1
      f) Vaccenic acid, 11 cis-18:1
      g) Linoleic acid, 9cis,12cis-18:2
      h) Eicosatrienoic acid, 8cis,11cis,14cis-20 :3
      i) Arachidonic acid, 5cis,8cis, 11cis,14cis-20 :4
      l) Trans isomers of arachidonic acid, mixture of mono-trans isomers of arachidonic acid: 5cis,8cis,11cis,14trans-20:4 + 5cis,8cis,11trans,14cis-20:4 + 5cis,8trans,11cis,14cis-20:4 + 5trans,8cis,11cis,14cis-20:4
      m) Eicosapentaenoic acid, 5cis,8cis,11cis,14cis,17cis-20:5
      n) Docosahexaenoic acid, 4cis,7cis,10cis,13cis,16cis,19cis-22:6
      o) Total trans fatty acids, TFA consisting of the sum of the trans geometrical isomers 18:1 and 20:4
      p) Total SFA consisting of the sum of saturated fatty acids 16:0 + 18:0
      q) Total MUFA consisting of the sum of monounsaturated fatty acids 16:1 + cis 18:1
      r) Total PUFA consisting of the sum of polyunsaturated fatty acids 18:2, 20:3, 20:4, 20:5, 22:6
      s) Ratio between Saturated Fatty Acids and Monounsaturated Fatty Acids, SFA/MUFA
      t) Ratio between the sum of omega-6 acids, linoleic acid + eicosatrienoic acid + arachidonic acid, and the sum of omega-3 acids eicosapentaenoic acid + docosahexaenoic acid, OMEGA-6/OMEGA-3
      u) Ratio between linoleic acid and stearic acid, EFA deficiency;

   - the measured values for each of the above said items are compared with the Standard values of the fatty acid contents for the same kind of cell and the percentage variation of the measured values, in respect of the corresponding inferior or superior limit of the Standard Range, is calculated;
   - the obtained value is multiplied by an arbitrary number representing the Basic Conversion Factor; and the so obtained absolute values are summed to represent the total percentage of unbalance $\Sigma$ FA;

- the minimum and maximum Standard values of each item is multiplied by an arbitrary number representing a Standard Conversion Factor and the two limit values are mediated giving the Σ mean FA value representing the contributions of the elements of the standard membrane fatty acid analysis;
- the all comprehensive index expressing the total membrane cell unbalance MUI is calculated according to the following formula:

$$MUI = \Sigma \, FA \times 100 / \, \Sigma \, mean \, FA$$

wherein the arbitrary number for step 3 is represented by the conversion factors reported in the following Tables 1 - 4:

TABLE 1

| FATTY ACIDS | Normal intervals (%rel.) | Conversion Factors BASIC | |
|---|---|---|---|
| | | -%var | +%var |
| Palmitic (16:0) | 17-27 | 1 | 23 |
| Palmitoleic (16:1) | 0.2 - 0.5 | 1 | 13 |
| Stearic (18:0) | 13-20 | 23 | 2 |
| Oleic (18:1) | 9-18 | 1.7 | 1 |
| Trans 18:1 (9t+11t-18:1) | <0.3 | 0 | 0 |
| Vaccenic (18:1) | 0.7-1.3 | 0 | 0 |
| Linoleic (18:2) (omega-6) | 9-16 | 23 | 2.3 |
| Eicosatrienoic (20:3)(omega-6) | 1.9-2.4 | 2 | 2 |
| Arachidonic (20:4)(omega-6) | 13-17 | 2 | 2.3 |
| Mono trans ARA | <0.3 | 0 | |
| EPA (20:5) (omega-3) | 0.5-0.9 | 2 | 13 |
| DHA (22:6) (omega-3) | 5-7 | 2 | 13 |
| Tot. saturated (SFA) | 30-45 | 13 | 23 |
| Tot. monounsaturated (MUFA) | 13-23 | | 13 |
| Tot. polyunsaturated (PUFA) | 28-39 | 2.3 | 2.3 |
| Tot. trans fatty acids (TFA) | <0.3 | 0 | 23 |
| SFA/MUFA ratio | 1.7-2 | 0 | 23 |
| Omega-6/ omega-3 ratio | 3.5-5.5 | 0 | 13 |
| EFA deficiency | >0.4 | 0 | 1 |

TABLE 2

| FATTY ACIDS | Conversion Factors CARDIO | |
|---|---|---|
| | -%var | +%var |
| Palmitic (16:0) | 1 | 35 |
| Palmitoleic (16:1) | 2 | 1 |
| Stearic (18:0) | 2 | 4 |
| Oleic (18:1) | 2 | 1.5 |
| Trans 18:1 (9t+11t-18:1) | 0 | 3 |

(continued)

| FATTY ACIDS | Conversion Factors CARDIO | |
|---|---|---|
| | -%var | +%var |
| **Vaccenic (18:1)** | 2 | 1 |
| **Linoleic (18:2)(omega-6)** | 2.5 | 3 |
| **Eicosatrienoic (20:3)(omega-6)** | 3 | 3 |
| **Arachidonic (20:4)(omega-6)** | 3 | 35 |
| **Mono trans ARA** | 1 | 3 |
| **EPA (20:5) (omega-3)** | 3 | 15 |
| **DHA (22:6)(omega-3)** | 3 | 1 |
| **Tot. saturated (SFA)** | 1.5 | 35 |
| **Tot. monounsaturated (MUFA)** | 35 | 1.5 |
| **Tot. polyunsaturated (PUFA)** | 3.5 | 1 |
| **Tot. trans fatty acids (TFA)** | 0 | 3 |
| **SFA/MUFA ratio** | 2 | 35 |
| **Omega-6/ omega-3 ratio** | 2.5 | 35 |
| **EFA deficiency** | 2 | 3 |

## TABLE 3

| FATTY ACIDS | Conversion Factors UFA deficit | |
|---|---|---|
| | -%var | + %var |
| Palmitic (16:0) | 1.5 | 3 |
| Palmitoleic (16:1) | 2.5 | 1 |
| Stearic (18:0) | 2.5 | 3 |
| Oleic (18:1) | 2 | 1 |
| Trans 18:1 (9t +11t-18:1) | 1 | 3 |
| Vaccenic (18:1) | 1 | 1 |
| Linoleic (18:2) (omega-6) | 3 | 15 |
| Eicosatrienoicc (20:3) (omega-6) | 3 | 3 |
| Arachidonic (20:4)(omega-6) | 2.5 | 35 |
| Mono trans ARA | 1 | 3 |
| EPA (20:5) (omaga-3) | 3.5 | 2 |
| DHA (22:6) (omega-3) | 3.5 | 2 |
| Tot. saturated (SFA) | 1 | 3 |
| Tot. monounsaturated (MUFA) | 2 | 1 |
| Tot. polyunsaturated (PUFA) | 3 | 1 |
| Tot. trans fatty acids (TFA) | 1 | 3 |
| SFA/MUFA ratio | 2 | 35 |
| Omega-6/ omega-3 ratio | 1.5 | 3 |

(continued)

| FATTY ACIDS | Conversion Factors UFA deficit | |
|---|---|---|
| | -%var | + %var |
| EFA deficiency | 3 | 1 |

**TABLE 4**

| FATTY ACIDS | Conversion Factors STRESS | |
|---|---|---|
| | -%var | + %var |
| Palmitic (16:0) | 1 | 3 |
| Palmitoleic (16:1) | 2.5 | 1 |
| Stearic (18:0) | 2 | 3 |
| Oleic (18:1) | 2 | 2 |
| Trans 18:1 (9t+11t-18:1) | 0 | 35 |
| vaccenic (18:1) | 1 | 2 |
| Linoleic (18:2) (omega-6) | 3 | 2 |
| Eicosatrienoic (20:3) (omega-6) | 3 | 2 |
| Arachidonic (20:4)(omega-6) | 2 | 35 |
| Mono trans ARA | 0 | 35 |
| EPA (20:5) (omega-3) | 35 | 1 |
| DHA (22:6) (omega-3) | 35 | 1 |
| Tot. saturated (SFA) | 1 | 3 |
| Tot. monounsaturated (MUFA) | 1 | 2 |
| Tot. polyunsaturated (PUFA) | 3 | 1 |
| Tot. trans fatty acids (TFA) | 1 | 35 |
| SFA/MUFA ratio | 1 | 35 |
| Omega-6/ omega-3 ratio | 15 | 35 |
| EFA deficiency | 3 | 1 |

2. Method according to Claim 1 wherein the MUI is coupled with a chromatic and/or symbolic indicator.

3. Method according to Claim 2 wherein the chromatic indicator is as reported in Table 5

TABLE 5 - Possible Combinations and corresponding chromatic indicators

| FATTY ACIDS | COMBINATIONS | | | |
|---|---|---|---|---|
| | green | yellow | red | red |
| Palmitic (16:0) | | | +%var | |
| Palmitoleic (16:1) | | +%var | | |
| Stearic (18:0) | | | -%var | |
| Oleic (18:1) | +%var | +%var | | |
| Trans 18:1 | | | | |

(continued)

| FATTY ACIDS | COMBINATIONS | | | |
|---|---|---|---|---|
| | green | yellow | red | red |
| Vaccenic (18:1) | | +%var | | |
| Linoleic (18:2) (omega-6) | | | | |
| Eicosatrienoic (20:3) (omega-6) | | -%var | | -%var |
| Arachidonic (20:4)(omega-6) | | | | +%var |
| Mono trans ARA | | | | |
| EPA (20:5) (omega-3) | | | | |
| DHA (22:6) (omega-3) | | | -%var | |
| Tot. saturated (SFA) | | | +%var | |
| Tot. monounsaturated (MUFA) | +%var | | | |
| Tot. polyunsaturated (PUFA) | | -%var | | +%var |
| Tot. trans fatty acids (TFA) | | | | +%var |
| SFA/MUFA ratio | | -%var | +%var | |
| Omega-6/ omega-3 ratio | | | | +%var |
| EFA deficiency | | | | |

**Patentansprüche**

1. Verfahren zur globalen Evaluierung des Status von Zellmembranen bezüglich deren Fettsäurekomponenten, wobei die Fettsäuren Membranphospholipide sind und die Zellen Blutzellen sind, wobei das Verfahren die nachfolgenden Schritte umfasst:

- Proben des Patientenblutes werden gemäß bekannten Techniken analysiert, um die Mengen der nachfolgenden 19 Positionen zu bestimmen:

a) Palmitinsäure (16:0)
b) Stearinsäure (18:0)
c) Palmitoleinsäure (9cis-16:1)
d) Ölsäure (9cis-18:1)
e) Elaidinsäure (9trans-18:1) + Trans-Vaccensäure, 11 trans-18:1, ausgedrückt zusammen als Trans-18:1
f) Vaccensäure, 11 cis-18:1
g) Leinölsäure, 9cis, 12cis-18:2
h) Eicosatriensäure, 8cis, 11 cis, 14cis-20:3
i) Arachidonsäure, 5cis, 8cis, 11 cis, 14cis-20:4
l) Transisomere von Arachidonsäure, Mischungen von Mono-Transisomeren von Arachidonsäure: 5cis, 8cis, 11 cis, 14trans-20:4 + 5cis, 8cis, 11 trans, 14cis-20:4 + 5cis, 8trans, 11 cis, 14cis-20:4 + 5trans, 8cis, 11 cis, 14cis-20:4
m) Eicosapentaensäure, 5cis, 8cis, 11 cis, 14cis, 17cis-20:5
n) Docosahexaensäure, 4cis, 7cis, 10cis, 13cis, 16cis, 19cis-22:6
o) Gesamtransfettsäuren, TFA bestehend aus der Summe von transgeometrischen Isomeren 18:1 und 20:4
p) Gesamt-SFA bestehend aus der Summe von gesättigten Fettsäuren 16:0 + 18:0
q) Gesamt-MUFA bestehend aus der Summe von monogesättigten Fettsäuren 16:1 + cis 18:1
r) Gesamt-PUFA bestehend aus der Summe von polyungesättigten Fettsäuren 18:2, 20:3, 20:4, 20:5, 22:6
s) Verhältnis zwischen gesättigten Fettsäuren und monogesättigten Fettsäuren, SFA/MUFA
t) Verhältnis zwischen der Summe von Omega-6-Säuren, Leinölsäure + Eicosatriensäure + Arachidonsäure und der Summe von Omega-3-Säuren Eicosapentaensäure + Docosahexaensäure, OMEGA-6/OMEGA-3
u) Verhältnis zwischen Leinölsäure und Stearinsäure, EFA-Defizienz,

- die gemessenen Werte für jede der vorstehenden Positionen werden mit den Standardwerten der Fettsäuremengen für dieselbe Art von Zellen verglichen und es werden die prozentualen Abweichungen der gemessenen Werte bezogen auf die entsprechende Unter- oder Obergrenze des Standardbereiches berechnet,

- der erhaltene Wert wird mit einer willkürlichen Zahl, welche den Grundumrechnungsfaktor wiedergibt, multipliziert und die so erhaltenen absoluten Werte werden summiert, um den Gesamtprozentsatz des Ungleichgewichts Σ FA wiederzugeben,

- die minimalen und maximalen Standardwerte von jeder Position werden mit einer zufälligen Zahl, welche den Standardumrechnungsfaktor wiedergibt, multipliziert und die beiden Grenzwerte werden gemittelt, um den Σ durchschnittlichen FA-Wert zu ergeben, welcher die Beiträge der Elemente der Standardmembran-Fettsäureanalyse wiedergibt,

- der alles umfassende Index ausgedrückt als die Gesamtmembranzahl-Unausgeglicheneinheit MUI wird gemäß der nachfolgenden Formel berechnet:

$$MUI = \sum FA \times 100/\sum \text{ durchschnittliches FA,}$$

wobei die willkürliche Zahl für den Schritt 3 durch die in den nachfolgenden Tabellen 1 bis 4 wiedergegebenen Umrechnungsfaktoren wiedergegeben sind:

Tabelle 1

| Fettsäuren | Normalintervalle (% rel.) | Grundumrechnungsfaktoren | |
|---|---|---|---|
| | | -%var | +%var |
| Palmitinsäure (16:0) | 17-27 | 1 | 2,3 |
| Palmitoleinsäure (16:1) | 0,2 - 0,5 | 1 | 1,3 |
| Sterinsäure (18:0) | 13-20 | 2,3 | 2 |
| Ölsäure (18:1) | 9-18 | 1,7 | 1 |
| Trans 18:1 (9t+11t-18:1) | <0,3 | 0 | 0 |
| Vaccensäure (18:1) | 0,7-1,3 | 0 | 0 |
| Leinölsäure (18:2) (omega-6) | 9-16 | 2,3 | 2,3 |
| Eicosatriensäure (20:3) (omega-6) | 1,9-2,4 | 2 | 2 |
| Arachidonsäure (20:4) (omega-6) | 13-17 | 2 | 2,3 |
| Mono-Trans ARA | <0,3 | 0 | |
| Eicosapentaensäure (20:5) (omega-3) | 0,5-0,9 | 2 | 1,3 |
| Docosahexaensäure (22:6) (omega-3) | 5-7 | 2 | 1,3 |
| Gesamtgesättigte (SFA) | 30-45 | 1,3 | 2,3 |
| Gesamtmonoungesättigte (MUFA) | 13-23 | | 1,3 |
| Gesamtpolyungesättigte (PUFA) | 28-39 | 2,3 | 2,3 |
| Gesamttransfettsäuren (TFA) | <0,3 | 0 | 2,3 |
| SFA/MUFA Verhältnis | 1,7-2 | 0 | 2,3 |
| Omega-6/Omega-3 Verhältnis | 3,5-5,5 | 0 | 1,3 |
| EFA Defizit | >0,4 | 0 | 1 |

Tabelle 2

| Fettsäuren | Umrechnungsfaktoren KARDIO | |
|---|---|---|
| | -%var | +%var |
| Palmitinsäure (16:0) | 1 | 3,5 |
| Palmitoleinsäure (16:1) | 2 | 1 |
| Sterinsäure (18:0) | 2 | 4 |
| Ölsäure (18:1) | 2 | 1,5 |
| Trans 18:1 (9t+11t-18:1) | 0 | 3 |
| Vaccensäure (18:1) | 2 | 1 |
| Leinölsäure (18:2) (omega-6) | 2,5 | 3 |
| Eicosatriensäure (20:3) (omega-6) | 3 | 3 |
| Arachidonsäure (20:4) (omega-6) | 3 | 3,5 |
| Mono-Trans ARA | 1 | 3 |
| Eicosapentaensäure (20:5) (omega-3) | 3 | 1,5 |
| Docosahexaensäure (22:6) (omega-3) | 3 | 1 |
| Gesamtgesättigte (SFA) | 1,5 | 3,5 |
| Gesamtmonoungesättigte (MUFA) | 3,5 | 1,5 |
| Gesamtpolyungesättigte (PUFA) | 3,5 | 1 |
| Gesamttransfettsäuren (TFA) | 0 | 3 |
| SFA/MUFA Verhältnis | 2 | 3,5 |
| Omega-6/Omega-3 Verhältnis | 2,5 | 3,5 |
| EFA Defizit | 2 | 3 |

Tabelle 3

| Fettsäuren | Umrechnungsfaktoren UFA Defizit | |
|---|---|---|
| | -%var | +%var |
| Palmitinsäure (16:0) | 1,5 | 3 |
| Palmitoleinsäure (16:1) | 2,5 | 1 |
| Sterinsäure (18:0) | 2,5 | 3 |
| Ölsäure (18:1) | 2 | 1 |
| Trans 18:1 (9t+11t-18:1) | 1 | 3 |
| Vaccensäure (18:1) | 1 | 1 |
| Leinölsäure (18:2) (omega-6) | 3 | 1,5 |
| Eicosatriensäure (20:3) (omega-6) | 3 | 3 |
| Arachidonsäure (20:4) (omega-6) | 2,5 | 3,5 |
| Mono-Trans ARA | 1 | 3 |
| Eicosapentaensäure (20:5) (omega-3) | 3,5 | 2 |
| Docosahexaensäure (22:6) (omega-3) | 3,5 | 2 |
| Gesamtgesättigte (SFA) | 1 | 3 |
| Gesamtmonoungesättigte (MUFA) | 2 | 1 |

(fortgesetzt)

| Fettsäuren | Umrechnungsfaktoren UFA Defizit | |
|---|---|---|
| | -%var | +%var |
| Gesamtpolyungesättigte (PUFA) | 3 | 1 |
| Gesamttransfettsäuren (TFA) | 1 | 3 |
| SFA/MUFA Verhältnis | 2 | 3,5 |
| Omega-6/Omega-3 Verhältnis | 1,5 | 3 |
| EFA Defizit | 3 | 1 |

Tabelle 4

| Fettsäuren | Umrechnungsfaktoren STRESS | |
|---|---|---|
| | -%var | +%var |
| Palmitinsäure (16:0) | 1 | 3 |
| Palmitoleinsäure (16:1) | 2,5 | 1 |
| Sterinsäure (18:0) | 2 | 3 |
| Ölsäure (18:1) | 2 | 2 |
| Trans 18:1 (9t+11t-18:1) | 0 | 3,5 |
| Vaccensäure (18:1) | 1 | 2 |
| Leinölsäure (18:2) (omega-6) | 3 | 2 |
| Eicosatriensäure (20:3) (omega-6) | 3 | 2 |
| Arachidonsäure (20:4) (omega-6) | 2 | 3,5 |
| Mono-Trans ARA | 0 | 3,5 |
| Eicosapentaensäure (20:5) (omega-3) | 3,5 | 1 |
| Docosahexaensäure (22:6) (omega-3) | 3,5 | 1 |
| Gesamtgesättigte (SFA) | 1 | 3 |
| Gesamtmonoungesättigte (MUFA) | 1 | 2 |
| Gesamtpolyungesättigte (PUFA) | 3 | 1 |
| Gesamttransfettsäuren (TFA) | 1 | 3,5 |
| SFA/MUFA Verhältnis | 1 | 3,5 |
| Omega-6/Omega-3 Verhältnis | 1,5 | 3,5 |
| EFA Defizit | 3 | 1 |

**2.** Verfahren nach Anspruch 1, wobei die MUI mit einem chromatischen und/oder symbolischen Indikator gekoppelt ist.

**3.** Verfahren nach Anspruch 2, wobei der chromatische Indikator wie in der Tabelle 5 wiedergegeben ist:

Tabelle 5 - Mögliche Kombinationen und entsprechende chromatische Indikatoren

| Fettsäuren | Kombinationen | | | |
|---|---|---|---|---|
| | Grün | Gelb | Rot | Rot |
| Palmitinsäure (16:0) | | | +%var | |

(fortgesetzt)

| Fettsäuren | Kombinationen | | | |
|---|---|---|---|---|
| | Grün | Gelb | Rot | Rot |
| Palmitoleinsäure (16:1) | | +%var | | |
| Sterinsäure (18:0) | | | +%var | |
| Ölsäure (18:1) | +%var | +%var | | |
| Trans 18:1 | | | | |
| Vaccensäure (18:1) | | +%var | | |
| Linolsäure (18:2) (omega-6) | | | | |
| Eicosatriensäure (20:3) (omega-6) | | -%var | | -%var |
| Arachidonsäure (20:4) (omega-6) | | | | +%var |
| Mono-Trans ARA | | | | |
| EPA (20:5) (omega-3) | | | | |
| DHA (22:6) (omega-3) | | | -%var | |
| Gesamtgesättigte (SFS) | | | +%var | |
| Gesamtmonoungesättigte (MUFA) | +%var | | | |
| Gesamtpolyungesättigte (PUFA) | | -%var | | +%var |
| Gesamttransfettsäure (TFA) | | | | +%var |
| SFA/MUFA Verhältnis | | -%var | +%var | |
| Omega-6/Omega-3 Verhältnis | | | | +%var |
| EFA Defizit | | | | |

**Revendications**

1. Procédé d'évaluation globale du statut des membranes cellulaires lié à leurs composants acides gras, dans lequel lesdits acides gras sont des phospholipides membranaires et lesdites cellules sont des cellules sanguines ; ledit procédé comprenant l'étape suivante :

    - des échantillons de sang prélevés sur des patients sont analysés selon des techniques connues en vue d'établir les quantités des 19 éléments suivants :

        a) acide palmitique (16:0)
        b) acide stéarique (18:0)
        c) acide palmitoléique (9cis-16:1)
        d) acide oléique (9cis-18:1)
        e) acide élaïdique (9trans-18:1) + acide trans-vaccénique, 11trans-18:1, exprimé ensemble comme trans-18:1
        f) acide vaccénique, 11cis-18:1
        g) acide linoléique, 9cis, 12cis-18:2
        h) acide éicosatriénoïque, 8cis, 11cis, 14cis-20:3
        i) acide arachidonique, 5cis, 8cis, 11cis, 14cis-20:4
        l) isomère trans de l'acide arachidonique, mélange d'isomères mono-trans d'acide arachidonique : 5cis, 8cis, 11cis, 14trans-20:4 + 5cis, 8cis, 11trans, 14cis-20:4 + 5cis, 8trans, 11cis, 14cis-20:4 + 5trans, 8cis, 11cis, 14cis-20:4
        m) acide éicosapentaénoïque, 5cis, 8cis, 11cis, 14cis, 17cis-20:5
        n) acide docosahexaénoïque, 4cis, 7cis, 10cis, 13cis, 16cis, 19cils-22:6
        o) acides trans gras totaux, TFA constitués de la somme des isomères trans géométriques 18:1 et 20:4

p) SFA totaux constitués de la somme des acides gras saturés 16:0 + 18:0

q) MUFA totaux constitués de la somme des acides gras mono-insaturés 16:1 + cis 18:1

r) PUFA totaux constitués de la somme des acides gras polyinsaturés 18:2, 20:3, 20:4, 20:5, 22:6

s) rapport des acides gras saturés sur les acides gras mono-insaturés, SFA/MUFA

t) rapport de la somme des acides oméga-6, acide linoléique + acide éicosatriénoïque + acide arachidonique, sur la somme des acides oméga-3 acide éicosapentaénoïque + acide docosahexaénoïque, OMEGA-6 / OMéGA-3

u) rapport de l'acide linoléique sur l'acide stéarique, insuffisance en EFA ;

- les valeurs mesurées pour chacun des éléments ci-dessus sont comparées aux valeurs standard des teneurs en acides gras pour la même sorte de cellules et le pourcentage de variation des valeurs mesurées, relativement à la limite inférieure ou supérieure correspondante de la plage standard, est calculé ;

- la valeur obtenue est multipliée par un nombre arbitraire représentant le facteur de conversion basique ; et les valeurs absolues ainsi obtenues sont totalisées pour représenter le pourcentage total de déséquilibre de $\Sigma$ FA ;

- les valeurs standard minimales et maximales de chaque élément sont multipliées par un nombre arbitraire représentant un facteur de conversion standard et une moyenne des deux valeurs limites est établie, donnant la valeur de la $\Sigma$ moyenne des FA représentant les contributions des éléments de l'analyse des acides gras standard de la membrane ;

- l'indice global, MUI, exprimant le déséquilibre cellulaire total de la membrane, est calculé selon la formule suivante :

$$MUI = \Sigma\ FA\ x\ 100/\Sigma\ moyenne\ FA$$

dans lequel le nombre arbitraire de l'étape 3 est représenté par les facteurs de conversion rapportés dans les tableaux 1 à 4 suivants :

Tableau 1

| ACIDES GRAS | INTERVALLES NORMAUX | Facteurs de conversion BASIQUES | |
| --- | --- | --- | --- |
| | | -%var | +%var |
| Palmitique (16:0) | 17-27 | 1 | 2,3 |
| Palmitoléique (16:1) | 0,2-0,5 | 1 | 1,3 |
| Stéarique (18:0) | 13-20 | 2,3 | 2 |
| Oléique (18:1) | 9-18 | 1,7 | 1 |
| Trans 18:1 (9t + 11t + 18:1) | <0,3 | 0 | 0 |
| Vaccénique (18:1) | 0,7-1,3 | 0 | 0 |
| Linoléique (18:1) (oméga-6) | 9-16 | 2,3 | 2,3 |
| Eicosatriénoïque (20:3) (oméga-6) | 1,9-2,4 | 2 | 2 |
| Arachidonique (20:4) (oméga-6) | 13-17 | 2 | 2,3 |
| ARA mono-trans | <0,3 | 0 | |
| EPA (20:5) (oméga-3) | 0,5-0,9 | 2 | 1,3 |
| DHA (22:6) (oméga-3) | 5-7 | 2 | 1,3 |
| Saturés totaux (SFA) | 30-45 | 1,3 | 2,3 |
| Mono-insaturés totaux (MUFA) | 13-23 | | 1,3 |
| Polyinsaturés totaux (PUFA) | 28-39 | 2,3 | 2,3 |
| Acides gras trans totaux (TFA) | <0,3 | 0 | 2,3 |
| Rapport SFA/MUFA | 1, 7-2 | 0 | 2,3 |

(suite)

| ACIDES GRAS | INTERVALLES NORMAUX | Facteurs de conversion BASIQUES | |
|---|---|---|---|
| | | -%var | +%var |
| Rapport oméga-6/oméga-3 | 3,5 - 5,5 | 0 | 1,3 |
| Insuffisanse en EFA | > 0,4 | 0 | 1 |

Tableau 2

| ACIDES GRAS | Facteurs de conversion CARDIO | |
|---|---|---|
| | -%var | +%var |
| Palmitique (16:0) | 1 | 3,5 |
| Palmitoléique (16:1) | 2 | 1 |
| Stéarique (18:0) | 2 | 4 |
| Oléique (18:1) | 2 | 1,5 |
| Trans 18:1 (91 + 11t + 18:1) | 0 | 3 |
| Vaccénique (18:1) | 2 | 1 |
| Linoléique (18:1) (oméga-6) | 2,5 | 3 |
| Eicosatriénoïque (20:3) (oméga-6) | 3 | 3 |
| Arachidonique (20:4) (oméga-6) | 3 | 3,5 |
| ARA mono-trans | 1 | 3 |
| EPA (20:5) (oméga-3) | 3 | 1,5 |
| DHA (22:6) (oméga-3) | 3 | 1 |
| Saturés totaux (SFA) | 1,5 | 3,5 |
| Mono-insaturés totaux (MUFA) | 3,5 | 1,5 |
| Polyinsaturés totaux (PUFA) | 3,5 | 1 |
| Acides gras trans totaux (TFA) | 0 | 3 |
| Rapport SFA/MUFA | 2 | 3,5 |
| Rapport oméga-6/oméga-3 | 2,5 | 3,5 |
| Insuffisance en EFA | 2 | 3 |

Tableau 3

| ACIDES GRAS | Facteurs de conversion Déficit en UVA | |
|---|---|---|
| | -%var | +%var |
| Palmitique (16:0) | 1,5 | 3 |
| Palmitoléique (16:1) | 2,5 | 1 |
| Stéarique (18:0) | 2,5 | 3 |
| Oléique (18:1) | 2 | 1 |
| Trans 18:1 (9t + 11t + 18:1) | 1 | 3 |
| Vaccénique (18:1) | 1 | 1 |
| Linoléique (18:1) (oméga-6) | 3 | 1,5 |

(suite)

| ACIDES GRAS | Facteurs de conversion Déficit en UVA | |
|---|---|---|
| | -%var | +%var |
| Eicosatriénoïque (20:3) (oméga-6) | 3 | 3 |
| Arachidonique (20:4) (oméga-6) | 2,5 | 3,5 |
| ARA mono-trans | 1 | 3 |
| EPA (20:5) (oméga-3) | 3,5 | 2 |
| DHA (22:6) (oméga-3) | 3,5 | 2 |
| Saturés totaux (SFA) | 1 | 3 |
| Mono-insaturés totaux (MUFA) | 2 | 1 |
| Polyinsaturés totaux (PUFA) | 3 | 1 |
| Acides gras trans totaux (TFA) | 1 | 3 |
| Rapport SFA/MUFA | 2 | 3,5 |
| Rapport oméga-6/oméga-3 | 1,5 | 3 |
| insuffisance en EFA | 3 | 1 |

Tableau 4

| ACIDES GRAS | Facteurs de conversion STRESS | |
|---|---|---|
| | -%var | +%var |
| Palmitique (16:0) | 1 | 3 |
| Palmitoléique (16:1) | 2,5 | 1 |
| Stéarique (18:0) | 2 | 3 |
| Oléique (18:1) | 2 | 2 |
| Trans 18:1 (9t + 11t + 18:1) | 0 | 3,5 |
| Vaccénique (18:1) | 1 | 2 |
| Linoléique (18:1) (oméga-6) | 3 | 2 |
| Eicosatriénoïque (20:3) (oméga-6) | 3 | 2 |
| Arachidonique (20:4) (oméga-6) | 2 | 3,5 |
| ARA mono-trans | 0 | 3,5 |
| EPA (20:5) (oméga-3) | 3,5 | 1 |
| DHA (22:6) (oméga-3) | 3,5 | 1 |
| Saturés totaux (SFA) | 1 | 3 |
| Mono-insaturés totaux (MUFA) | 1 | 2 |
| Polyinsaturés totaux (PUFA) | 3 | 1 |
| Acides gras trans totaux (TFA) | 1 | 3,5 |
| Rapport SFA/MUFA | 1 | 3,5 |
| Rapport oméga-6/oméga-3 | 1,5 | 3,5 |
| Insuffisance en EFA | 3 | 1 |

2. Procédé selon la revendication 1, dans lequel le MUI est couplé à un indicateur chromatique et/ou symbolique.

3. Procédé selon la revendication 2, dans lequel l'indicateur chromatique est tel que rapporté dans le tableau 5

Tableau 5 - Combinaisons possibles et indicateurs chromatiques correspondants

| ACIDES GRAS | COMBINAISONS | | | |
|---|---|---|---|---|
| | vert | jaune | rouge | rouge |
| Palmitique (16:0) | | | +%var | |
| Palmitoléique (16:1) | | +%var | | |
| Stéarique (18:0) | | | -%var | |
| Oléique (18:1) | +%var | +%var | | |
| Trans 18:1 | | | | |
| Vaccénique (18:1) | | +%var | | |
| Linoléique (18:1) (oméga-6) | | | | |
| Eicosatriénoïque (20:3) (oméga-6) | | -%var | | -%var |
| Arachidonique (20:4) (oméga-6) | | | | +%var |
| ARA mono-trans | | | | |
| EPA (20:5) (oméga-3) | | | | |
| DHA (22:6) (oméga-3) | | | -%var | |
| Saturés totaux (SFA) | | | +%var | |
| Mono-insaturés totaux (MUFA) | +%var | | | |
| Polyinsaturés totaux (PUFA) | | -%var | | +%var |
| Acides gras trans totaux (TFA) | | | | +%var |
| Rapport SFA/MUFA | | -%var | +%var | |
| Rapport oméga-6/oméga-3 | | | | -%var |
| insuffisance en EFA | | | | |

Fig.1

**EP 2 356 468 B1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IT 1327120
- US 2005009005 A
- US 5075101 A

**Non-patent literature cited in the description**

- **MAXFIELD F.R. et al.** Role of cholesterol and lipid organisation in disease. *Nature,* 2005, vol. 438, 612
- **BLIGH E.G. et al.** A rapid method of total lipid extraction and purification. *Can. J. Biochem. Physiol.,* 1959, vol. 37, 911
- **KRAMER J. K. G. et al.** Evaluating acid and base catalysts in the methylation of milk and rumen fatty acids with special emphasis on conjugated dienes and total trans fatty acids. *Lipids,* 1997, vol. 32, 1219
- **ABDELOUAHID SAMADI et al.** *JAOCS,* 2004, vol. 81 (8), 753-758
- **FERRERI et al.** *Lipids,* 2005, vol. 40 (7), 661-667
- **GUNSTONE F.D.** Fatty acids and Lipid Chemistry. Aspen Publishers, 1999
- **LAURITZEN L. et al.** The essentiality of long chain n-3 fatty acids in relation to development and function of the brain and retina. *Progr. Lipid Res.,* 2001, vol. 40, 1